# EUROPÄISCHE PATENTSCHRIFT

(11) **EP 4 074 686 B1**
(45) Veröffentlichungstag und Bekanntmachung des Hinweises auf die Patenterteilung: **31.05.2023**
(21) Anmeldenummer: 21168829.6
(22) Anmeldetag: 16.04.2021
(51) Int. Cl.: C07C 37/11, C07C 39/15, C07C 37/84

(54) **VERFAHREN ZUR HERSTELLUNG VON BIPHENYL-2,2'-DIOLEN**
METHOD FOR THE PREPARATION OF BIPHENYL-2,2-DIOLS
PROCÉDÉ DE PRODUCTION DE BIPHÉNYL-2,2'-DIOLS

(43) Veröffentlichungstag der Anmeldung: 19.10.2022
(73) Patentinhaber: Evonik Operations GmbH, 45128 Essen (DE)
(72) Erfinder: FRIDAG, Dirk, 45721 Haltern am See (DE); SALE, Ana Chiara, 45657 Recklinghausen (DE); FRANKE, Robert, 45772 Marl (DE); BRÄCHER, Alexander, 45721 Haltern am See (DE); KNOSSALLA, Johannes, 46514 Gahlen (DE)
(74) Vertreter: Evonik Patent Association

(56) Entgegenhaltungen:
- WO-A1-99/46227

## Beschreibung

Die Erfindung betrifft ein Verfahren zur Herstellung von Biphenyl-2,2'-diolen. Die Phenylringe können hierbei neben den OH-Gruppen auch noch weitere Substituenten aufweisen.

In EP 2906571 B1 wird auf den Seiten 11 - 12 die Synthese von 2,2'-Bis(3,5-dimethylphenol) (**1**) beschrieben.

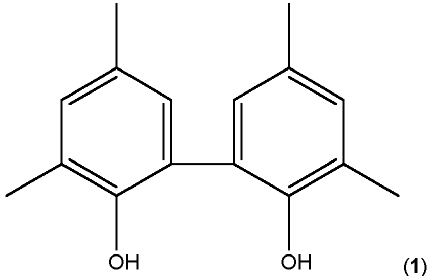

Hierbei wird 2,4-Dimethylphenol unter Zugabe von FeSO₄ und Na₂S₂O₈ umgesetzt. Das Produkt (1) wird mit einer Ausbeute von 69% erhalten. In der WO 99/46227 wird eine Oxidation von Phenolen mit Wasserstoffperoxid beschrieben, die jedoch zu einem negativen Ergebnis führt.

Der Erfindung lag die Aufgabe zugrunde, ein Verfahren bereitzustellen, mit welchen Biphenyl-2,2'-diole mit gesteigerter Ausbeute hergestellt werden können.

Die Aufgabe wird gelöst durch ein Verfahren nach Anspruch 1.

Verfahren umfassend die Verfahrensschritte:
a) Zugabe von Natrium-n-dodecylsulfat,
b) Zugabe einer Base,
c) Zugabe eines Phenols der Formel (**I**): 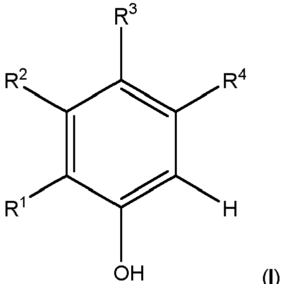
   wobei R¹, R², R³, R⁴ ausgewählt sind aus:

      -H, -(C1-C12)-Alkyl, -O-(C₁-C₁₂)-Alkyl, -(C6-C20)-Aryl, -O-(C₆-C₂₀)-Aryl;
   wobei die genannten Alkylgruppen und Arylgruppen wie folgt substituiert sein können:
      substituierte -(C₁-C₁₂)-Alkylgruppen, substituierte -O-(C₁-C₁₂)-Alkyl, substituierte -(C₆-C₂₀)-Aryl, substituierte -O-(C₆-C₂₀)-Aryl können einen oder mehrere Substituenten aufweisen; die Substituenten sind unabhängig voneinander ausgewählt aus: -(C₁-C₁₂)-Alkyl, -O-(C₁-C₁₂)-Alkyl, Halogen,
d) Erwärmen des Reaktionsgemisches aus a) bis c),
e) Zugabe von Wasserstoffperoxid zum Reaktionsgemisch,
f) Abkühlen des Reaktionsgemisches, so dass das Produkt (**II**) ausfällt:

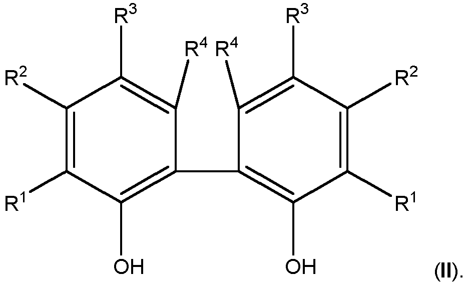

In einer Variante des Verfahrens sind R¹, R², R³, R⁴ ausgewählt aus:

-H, -(C₁-C₁₂)-Alkyl, -O-(C₁-C₁₂)-Alkyl.

In einer Variante des Verfahrens stehen R² und R⁴ für -H.

In einer Variante des Verfahrens sind R¹ und R³ ausgewählt aus:

-(C₁-C₁₂)-Alkyl, -O-(C₁-C₁₂)-Alkyl.

In einer Variante des Verfahrens stehen R¹ und R³ für -(C₁-C₁₂)-Alkyl.

In einer Variante des Verfahrens weist das Phenol (**I**) die Struktur (**3**) auf:

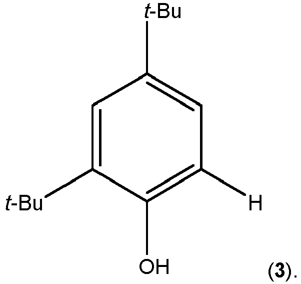

In einer Variante des Verfahrens ist die Base in Verfahrensschritt b) Natriumhydroxid.

In einer Variante des Verfahrens erfolgt das Erwärmen in Verfahrensschritt d) auf eine Temperatur im Bereich von 50 °C bis 100 °C.

In einer Variante des Verfahrens erfolgt das Erwärmen in Verfahrensschritt d) auf eine Temperatur im Bereich von 65 °C bis 95 °C.

In einer Variante des Verfahrens erfolgt die Zugabe von Wasserstoffperoxid in Verfahrensschritt e) durch Zutropfen einer Wasserstoffperoxidlösung.

In einer Variante des Verfahrens wird die in Verfahrensschritt d) eingestellte Temperatur währen der Zugabe des Wasserstoffperoxids gehalten.

In einer Variante des Verfahrens wird die in Verfahrensschritt d) eingestellte Temperatur nach der Zugabe des Wasserstoffperoxids für einen Zeitraum im Bereich von 10 Minuten bis 2 Stunden gehalten.

In einer Variante des Verfahrens umfasst dieses den zusätzlichen Verfahrensschritt f2):
f2) aktives Abkühlen des Reaktionsgemisches.

In einer Variante des Verfahrens erfolgt das aktive Abkühlen mit Hilfe eines Eisbades.

In einer Variante des Verfahrens umfasst dieses den zusätzlichen Verfahrensschritt g):
g) Waschen des ausgefallenen Produktes mit Wasser.

Im Folgenden soll die Erfindung anhand eines Ausführungsbeispieles näher erläutert werden.

### Synthese 3,3',5,5'-Tetra-tert-butylbiphenyl-2,2'-diol (2)

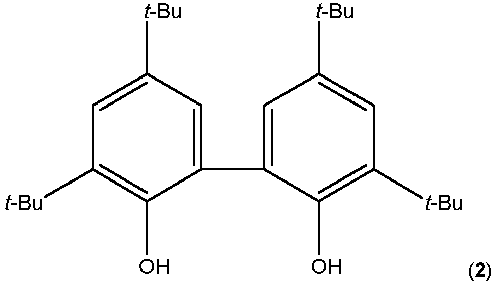

Zu einer Lösung aus 1,2 g (4 mmol) Natrium-n-dodecylsulfat in 400 ml VE-Wasser werden nacheinander unter Rühren 64 g (1,6 mol) Natriumhydroxid und 166,72 g (0,8 mol) 2,4-Di-tert-butylphenol (**3**) gegeben. Anschließend wird die Lösung auf 85 °C erwärmt. Dann werden innerhalb von 90 min 58,4 ml (0,674 mol) einer 35-igen Wasserstoffperoxidlösung zugetropft, wobei die Reaktionstemperatur zwischen 85 °C und 90 °C gehalten wird. Dies wurde erreicht indem die Badtemperatur auf 70 °C abgesenkt wurde da die Reaktion exotherm ist. Nach dem Zutropfen wird für weitere 30 min bei 85 °C gerührt. Danach wurde das Ölbad auf 22 °C gestellt und die Reaktionslösung kühlte ca. 2 h ab. Anschließen wurde diese noch 1 h in ein Eisbad gestellt. Die Reaktionslösung wurde danach filtriert und der Rückstand zweimal mit 40 ml Wasser gewaschen. Der erhaltene Feststoff wurde 18 h im Vakuum getrocknet. Die Ausbeute betrug 166,3 g (99,2 %).

Der durchgeführte Versuch belegt, dass die gestellte Aufgabe durch das erfindungsgemäße Verfahren gelöst wird.

## Patentansprüche

1. Verfahren umfassend die Verfahrensschritte:
a) Zugabe von Natrium-n-dodecylsulfat,
b) Zugabe einer Base,
c) Zugabe eines Phenols der Formel (**I**): 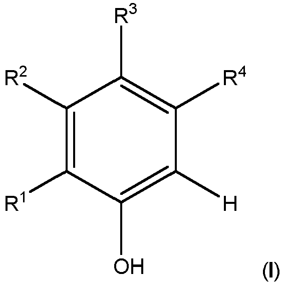
wobei R¹, R², R³, R⁴ ausgewählt sind aus:
-H, -(C1-C12)-Alkyl, -O-(C₁-C₁₂)-Alkyl, -(C6-C20)-Aryl, -O-(C₆-C₂₀)-Aryl;
wobei die genannten Alkylgruppen und Arylgruppen wie folgt substituiert sein können:
substituierte -(C₁-C₁₂)-Alkylgruppen, substituierte -O-(C₁-C₁₂)-Alkyl, substituierte -(C₆-C₂₀)-Aryl, substituierte -O-(C₆-C₂₀)-Aryl können einen oder mehrere Substituenten aufweisen; die Substituenten sind unabhängig voneinander ausgewählt aus: -(C₁-C₁₂)-Alkyl, -O-(C₁-C₁₂)-Alkyl, Halogen,
d) Erwärmen des Reaktionsgemisches aus a) bis c),
e) Zugabe von Wasserstoffperoxid zum Reaktionsgemisch,
f) Abkühlen des Reaktionsgemisches, so dass das Produkt (**II**) ausfällt:

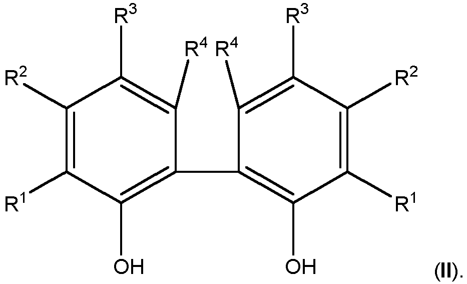

2. Verfahren nach Anspruch 1,
wobei R¹, R², R³, R⁴ ausgewählt sind aus:
-H, -(C₁-C₁₂)-Alkyl, -O-(C₁-C₁₂)-Alkyl.

3. Verfahren nach einem der Ansprüche 1 oder 2,
wobei R² und R⁴ für -H stehen.

4. Verfahren nach einem der Ansprüche 1 bis 3,
wobei R¹ und R³ ausgewählt sind aus:
-(C₁-C₁₂)-Alkyl, -O-(C₁-C₁₂)-Alkyl.

5. Verfahren nach einem der Ansprüche 1 bis 4,
wobei R¹ und R³ für -(C₁-C₁₂)-Alkyl stehen.

6. Verfahren nach einem der Ansprüche 1 bis 5,
wobei das Phenol (**I**) die Struktur (3) aufweist:

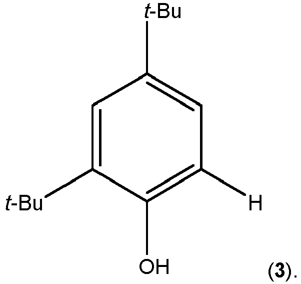

7. Verfahren nach einem der Ansprüche 1 bis 6,
wobei die Base in Verfahrensschritt b) Natriumhydroxid ist.

8. Verfahren nach einem der Ansprüche 1 bis 7,
wobei das Erwärmen in Verfahrensschritt d) auf eine Temperatur im Bereich von 50 °C bis 100 °C erfolgt.

9. Verfahren nach einem der Ansprüche 1 bis 8,
wobei die Zugabe von Wasserstoffperoxid in Verfahrensschritt e) durch Zutropfen einer Wasserstoffperoxidlösung erfolgt.

10. Verfahren nach einem der Ansprüche 1 bis 9,
wobei die in Verfahrensschritt d) eingestellte Temperatur währen der Zugabe des Wasserstoffperoxids gehalten wird.

11. Verfahren nach einem der Ansprüche 1 bis 10,
wobei die in Verfahrensschritt d) eingestellte Temperatur nach der Zugabe des Wasserstoffperoxids für einen Zeitraum im Bereich von 10 Minuten bis 2 Stunden gehalten wird.

12. Verfahren nach einem der Ansprüche 1 bis 11,
umfassend den zusätzlichen Verfahrensschritt f2):
f2) aktives Abkühlen des Reaktionsgemisches.

13. Verfahren nach Anspruch 12,
wobei das aktive Abkühlen mit Hilfe eines Eisbades erfolgt.

14. Verfahren nach einem der Ansprüche 1 bis 13,
umfassend den zusätzlichen Verfahrensschritt g):
g) Waschen des ausgefallenen Produktes mit Wasser.

## Claims

1. Process comprising the process steps of:
a) adding sodium n-dodecyl sulfate,
b) adding a base,
c) adding a phenol of the formula (**I**): 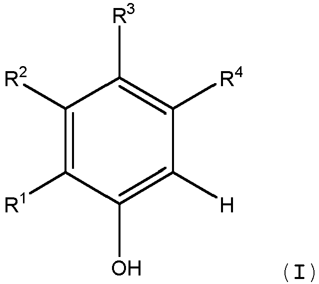
where R¹, R², R³, R⁴ are selected from:
-H, -(C₁-C₁₂)-alkyl, -O-(C₁-C₁₂)-alkyl, -(C₆-C₂₀)-aryl, -O-(C₆-C₂₀)-aryl;
wherein the alkyl groups and aryl groups cited may be substituted as follows:
substituted -(C₁-C₁₂)-alkyl groups, substituted -O-(C₁-C₁₂) -alkyl, substituted -(C₆-C₂₀)-aryl, substituted -O-(C₆-C₂₀)-aryl may have one or more substituents; the substituents are each independently selected from: -(C₁-C₁₂) -alkyl, -O-(C₁-C₁₂)-alkyl, halogen,
d) heating the reaction mixture of a) to c),
e) adding hydrogen peroxide to the reaction mixture,
f) cooling the reaction mixture so that the product (**II**) precipitates:

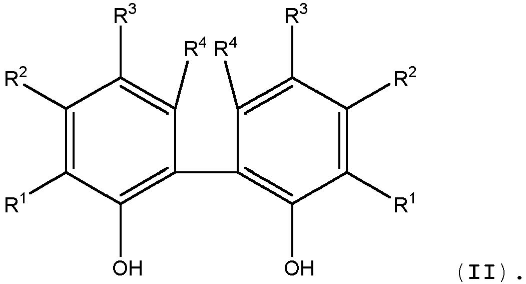

2. Process according to Claim 1,
where R¹, R², R³, R⁴ are selected from:
-H, -(C₁-C₁₂)-alkyl, -O-(C₁-C₁₂)-alkyl .

3. Process according to either of Claims 1 and 2,
where R² and R⁴ are each -H.

4. Process according to any of Claims 1 to 3,
where R¹ and R³ are selected from:
-(C₁-C₁₂)-alkyl, -O-(C₁-C₁₂)-alkyl.

5. Process according to any of Claims 1 to 4,
where R¹ and R³ are -(C₁-C₁₂)-alkyl.

6. Process according to any of Claims 1 to 5,
wherein the phenol (**I**) has the structure (**3**):

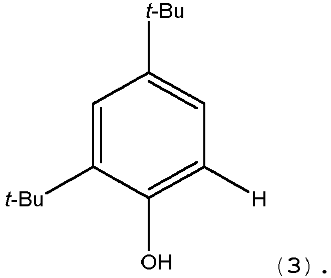

7. Process according to any of Claims 1 to 6,
wherein the base in process step b) is sodium hydroxide.

8. Process according to any of Claims 1 to 7,
wherein the heating in process step d) is carried out at a temperature in the range from 50°C to 100°C.

9. Process according to any of Claims 1 to 8,
wherein the addition of hydrogen peroxide in process step e) is carried out by dropwise addition of a hydrogen peroxide solution.

10. Process according to any of Claims 1 to 9,
wherein the temperature set in process step d) is maintained during the addition of hydrogen peroxide.

11. Process according to any of Claims 1 to 10,
wherein the temperature set in process step d) is maintained over a period in the range from 10 minutes to 2 hours after the addition of hydrogen peroxide.

12. Process according to any of Claims 1 to 11,
comprising the additional process step f2):
f2) active cooling of the reaction mixture.

13. Process according to Claim 12,
wherein the active cooling is effected with the aid of an ice bath.

14. Process according to any of Claims 1 to 13,
comprising the additional process step g):
g) washing the precipitated product with water.

## Revendications

1. Procédé comprenant les étapes de procédé :
a) ajout de n-dodécylsulfate de sodium,
b) ajout d'une base,
c) ajout un phénol de formule (I) : 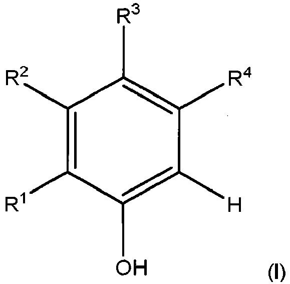
R¹, R², R³, R⁴ étant choisis parmi : -H, -(C₁-C₁₂)-alkyle, -O-(C₁-C₁₂)-alkyle, -(C₆-C₂₀)-aryle, -O-(C₆-C₂₀)-aryle ;
les groupes alkyle et les groupes aryle mentionnés pouvant être substitués comme suit : les groupes - (C₁-C₁₂)-alkyle substitué, -O-(C₁-C₁₂)-alkyle substitué, -(C₆-C₂₀)-aryle substitué, -O-(C₆-C₂₀)-aryle substitué peuvent présenter un ou plusieurs substituants ; les substituants sont choisis indépendamment les uns des autres parmi : -(C₁-C₁₂)-alkyle, -O-(C₁-C₁₂)-alkyle, halogène,
d) chauffage du mélange réactionnel de a) à c),
e) ajout de peroxyde d'hydrogène au mélange réactionnel,
f) refroidissement du mélange réactionnel de telle sorte que le produit (II) précipite :

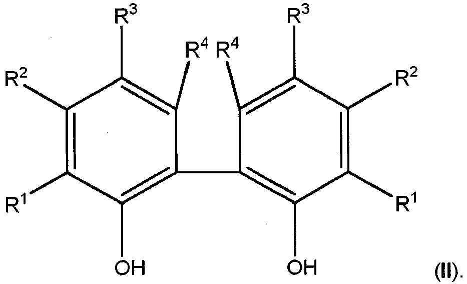

2. Procédé selon la revendication 1, R¹, R², R³, R⁴ étant choisis parmi : -H, -(C₁-C₁₂)-alkyle, -O-(C₁-C₁₂)-alkyle.

3. Procédé selon l'une quelconque des revendications 1 ou 2, R² et R⁴ représentant -H.

4. Procédé selon l'une quelconque des revendications 1 à 3, R¹ et R³ étant choisis parmi : -(C₁-C₁₂)-alkyle, -O-(C₁-C₁₂)-alkyle.

5. Procédé selon l'une quelconque des revendications 1 à 4, R¹ et R³ représentant -(C₁-C₁₂)-alkyle.

6. Procédé selon l'une quelconque des revendications 1 à 5, le phénol (I) présentant la structure (3) :

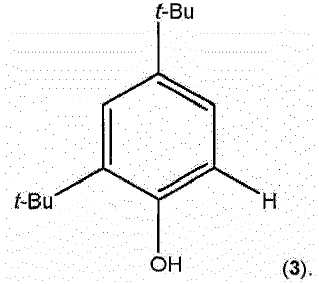

7. Procédé selon l'une quelconque des revendications 1 à 6, la base dans l'étape de procédé b) étant l'hydroxyde de sodium.

8. Procédé selon l'une quelconque des revendications 1 à 7, le chauffage dans l'étape de procédé d) étant réalisé à une température dans la plage de 50 °C à 100 °C.

9. Procédé selon l'une quelconque des revendications 1 à 8, l'ajout de peroxyde d'hydrogène dans l'étape de procédé e) ayant lieu par addition goutte à goutte d'une solution de peroxyde d'hydrogène.

10. Procédé selon l'une quelconque des revendications 1 à 9, la température réglée dans l'étape de procédé d) étant maintenue pendant l'ajout du peroxyde d'hydrogène.

11. Procédé selon l'une quelconque des revendications 1 à 10, la température réglée dans l'étape de procédé d) étant maintenue, après l'ajout du peroxyde d'hydrogène, pendant une période de temps dans la plage de 10 minutes à 2 heures.

12. Procédé selon l'une quelconque des revendications 1 à 11, comprenant l'étape de procédé supplémentaire f2) :
f2) refroidissement actif du mélange réactionnel.

13. Procédé selon la revendication 12, le refroidissement actif ayant lieu à l'aide d'un bain de glace.

14. Procédé selon l'une quelconque des revendications 1 à 13, comprenant l'étape de procédé supplémentaire g) :
g) lavage du produit précipité à l'eau.
